# EUROPEAN PATENT SPECIFICATION

(11) **EP 4 129 182 B1**
(45) Date of publication and mention of the grant of the patent: **24.09.2025**
(21) Application number: 21189602.2
(22) Date of filing: 04.08.2021
(51) Int. Cl.: A61B 6/00, A61B 5/055, A61B 8/08, G06T 7/30, A61B 5/00, A61B 5/06, A61B 90/00, A61B 34/10, A61B 34/20, A61B 8/00

(54) **TECHNIQUE FOR REAL-TIME VOLUMETRIC IMAGING FROM MULTIPLE SOURCES DURING INTERVENTIONAL PROCEDURES**
TECHNIK ZUR VOLUMETRISCHEN ECHTZEIT-ABBILDUNG AUS MEHREREN QUELLEN WÄHREND EINGRIFFSVERFAHREN
TECHNIQUE D'IMAGERIE VOLUMÉTRIQUE EN TEMPS RÉEL À PARTIR DE SOURCES MULTIPLES LORS DE PROCÉDURES D'INTERVENTION

(43) Date of publication of application: 08.02.2023
(73) Proprietor: Siemens Healthineers AG, 91301 Forchheim (DE)
(72) Inventor: QIU, Feng, Pennington, 08534 (US); PETKOV, Kaloian, Lawrenceville, 08648 (US); YU, Daphne, Yardley, 19067 (US); SOWRIRAJAN, Anthony Dass, Princeton Junction, 08550 (US)
(74) Representative: Schwarz, Claudia

(56) References cited:
- US-A1- 2016 030 008
- US-A1- 2018 235 701
- VICHOT FLORIAN ET AL: "Cardiac Interventional Guidance using Multimodal Data Processing and Visualisation: medInria as an Interoperability Platform", 2 January 2012 (2012-01-02), XP055875761, Retrieved from the Internet <URL:https://www.researchgate.net/profile/Maxime-Sermesant/publication/245022045_Cardiac_Interventional_Guidance_using_Multimodal_Data_Processing_and_Visualisation_medInria_as_an_Interoperability_Platform/links/562a1e2208aef25a243fc843/Cardiac-Interventional-Guidance-using-Multimodal-Data-Processing-and-> [retrieved on 20211228]

## Description

### FIELD OF THE INVENTION

The present disclosure relates to a technique for real-time volumetric imaging from multiple medical imaging sources during interventional procedures. More specifically, the technique relates to efficiently combining data from multiple medical imaging sources.

### TECHNICAL BACKGROUND

In interventional radiology, medical images, e.g., from X-ray fluoroscopy, ultrasound and computed tomography, are used to guide instruments such as catheters, stents and balloons through blood vessels or other pathways to treat diseases percutaneously. The interventional radiology procedures are conventionally less invasive and provide faster patient recovery than traditional surgery.

However, precision and clarity from the image guidance are essential to the success of such minimally invasive procedures. In many cases, the patients are scanned by high-resolution three-dimensional (3D) modalities (e.g., using computed tomography, CT, or magnetic resonance imaging, MRI) before the interventional procedure for planning. During the interventional procedure, the same high resolution 3D volume(s) may be registered with the real-time patient anatomy and with the live instruments to provide a 3D anatomical context.

The document *"*Cardiac Interventional Guidance using Multimodal Data Processing and Visualisation: medInria as an Interoperability Platform" by F. Vichot et al., 14 August 2021, describes a free medical imaging software used for pre-operative preparation for cardiac interventions. A CT-scan may be combined with two different types of meshes, which is denoted as multimodal integration. A "composite dataset" may use a ZIP archive to comprise a sequence of meshes as well as regular text storing metadata or annotations. A "Registration Programming Interface" (RPI) is introduced as a registration algorithm providing a generic API.

The patient may in addition be scanned under other real time modalities such as ultrasound and fluoroscopy during the procedure. In these cases, realistic visualizing of any tissue or segmented structures obtained from the 3D images together with the real-time instruments and images can provide improved visual guidance due to an improved understanding of the relation between structures and the instrument.

The document US 2016/0030008 A1 describes a method for co-displaying ultrasound information registered to an x-ray image. The ultrasound image and the x-ray image are each registered to a non-ultrasound image, e.g., a CT image, by a corresponding second and first transformation, and registering the ultrasound image to the x-ray image uses a third transformation based on the first and second transformation.

The document US 2018/0235701 A1 describes a multi-modality imaging method for guidance during an intervention using pre-operative planning annotations. During the intervention, ultrasound is used, and pre-operative imaging modalities comprise CT, x-ray fluoroscopy or MRI. Planning annotations from the pre-operative 3D image may be overlayed on the real-time ultrasound image, with the pre-operative 3D image and the ultrasound image being co-aligned or registered for suitable positioning of the annotations.

Ambient occlusion (AO) and subsurface scattering (SSS) are two 3D volume rendering techniques that can provide improved depth perception and realism.

In *"*Advanced Real-Time Rendering in 3D Graphics and Games", Chapter 9 Fast Approximations for Global Illumination on Dynamic Scenes, SIGGRAPH 2006 a signed distance field (SDF) is used for rendering a surface curvature in meshes using an AO effect.

In *"*Real-time Approximations to Subsurface Scattering", GPU Gems, Addison-Wesley Professional: 263-278, 2004*,* a distance of light traveling from a surface through a material to an inner point is estimated. The estimation assumes that the material inside the object is uniform, and a point light source or directional light source is used. The method is hence not suitable for images of a (e.g., part of a) human body. The estimation further does not consider diffusion of light when passing through the object.

Ropinski et al. in "Interactive Volume Rendering with Dynamic Ambient Occlusion and Color Bleeding", Comput. Graph. Forum. 27., 567-576 (2008*), and by* Kroes et al. in "Smooth Probabilistic Ambient Occlusion for Volume Rendering" (2018*)* describe local histograms or neighborhoods for local occlusions as interactive volumetric AO methods, without, however, taking into account a global AO.

A drawback of the conventional techniques is that they require large amounts of computational resources and samples, especially in the presence of multiple medical imaging sources, such as 3D volume(s), instruments, segmentation, to render a volumetric image. Hence, conventionally the rendering performance does not achieve a sufficiently low latency for guidance during the interventional procedure.

### SUMMARY

Accordingly, there is a need for a real-time volumetric rendering technique that efficiently combines data originating from multiple medical imaging sources during an interventional procedure. Alternatively, or in addition, there is a need for a technique that is cost efficient in terms of computing resources and/or that significantly reducing a latency of rendering a volumetric image based on the combined data.

As to a first aspect, a device for real-time volumetric imaging from multiple medical imaging sources of a part of a body during an interventional procedure according to claim 1 is provided.

The multiple medical imaging sources may be configured to provide data of the same part of the (e.g., human or animal) body, also denoted as the body of a patient. Alternatively, or in addition, the part of the body may comprise (e.g., part of) an organ, a bone, a vessel and/or soft tissue, e.g., in relation to a specific patient.

The device may be connected to any one of the medical imaging sources by a wired connection and/or by a wireless connection. The first data with the lowest temporal volatility may be obtained and/or acquired prior to the interventional procedure. E.g., an organ (e.g., comprising a liver and/or kidney) segmentation may be performed prior to the interventional procedure, e.g., using computed tomography (CT) and/or magnetic resonance imaging (MRI). The CT data and/or the MRI data may be distance transformed to obtain a binary mask, from which the organ (e.g., the liver and/or kidney) segmentation is constructed.

Alternatively, or in addition, the (N+1)-st data with the highest temporal volatility may be obtained and/or acquired during the interventional procedure, e.g., from a medical instrument used for the invention (for example a needle in an endoscopic intervention) and/or operated by a medical practitioner. Further or in addition, the (N+1)-st data may be obtained from a camera mounted on **(e.g.,** the tip of) a medical instrument inserted into a hollow structure **(e.g.,** a blood vessel and/or bladder) of the body during the interventional procedure, **e.g.,** during an endoscopic intervention.

Alternatively, or in addition, the (N+1)-st or any other data may comprise data obtained from **(e.g.,** X-ray) fluorescence and/or ultrasound imaging. The (e.g., X-ray) fluorescence and/or ultrasound imaging may be obtained and/or acquired during the interventional procedure.

By iteratively combining the data according to their respective temporal volatilities, a computational cost for outputting the real-time volumetric image data set (also denoted as real-time volumetric visualization data set) based on multiple medical imaging sources may be reduced. For example, the combining of the first and second data into the first intermediary data set is only performed if the second data are updated. As the third data are updated more frequently than the first data and the second data, the second intermediary data set is updated more frequently than the first intermediary data set. As the first intermediary data set does not require to be updated any time the third data are updated, computational costs and/or costs in terms of storage space are reduced. Alternatively, or in addition, a latency of obtaining a real-time volumetric image data set is reduced. Alternatively, on addition, a performance of outputting the real-time volumetric image data set based on multiple medical imaging sources may be stabilized.

By the inventive technique, real-time volumetric imaging during the interventional procedure allows the user (e.g., a medical practitioner) to reliably keep track of the location and/or orientation, where the intervention is performed, and/or allow to alert the user, in particular in case of deviations from a planned location and/or orientation of the intervention.

The data from at least one or each of the multiple medical imaging sources may be based on measurements and/or determinations of an intensity of light per position and/or per pixel. The measurements and/or determinations of the intensity of light per position and/or per pixel may be based on ambient light as a light source, e.g., emitting light rays observed and/or captured at the at least one or each of the multiple medical imaging sources (e.g., comprising at least one medical imaging source within the body during the interventional procedure and/or at least one medical imaging source outside of the body). The ambient light may be emitted from a headlight of a clinical practitioner operating at least one of the multiple medical imaging sources. Alternatively, or in addition, the ambient light may be emitted from a light source installed in a room, e.g., an overhead light.

Iteratively combining the received data may alternatively be denoted as and/or may comprise hierarchically reconstructing the received data. The hierarchy may refer to ordering the data according to the predefined criterion indicative of the temporal volatility.

The real-time volumetric image data set may be based on and/or may include curvature information (e.g., as to a vascular wall and/or a surface of an organ) comprised in and/or derived from the iteratively combined final data set.

The real-time volumetric image data set may be indicative and/or may comprise a directional view. A direction of the directional view may comprise at least one of anterior, posterior, left or right. The direction may be relative to the (e.g., human) body. E.g., anterior may relate to a view towards a frontal direction of the body, in particular comprising the face of the patient.

A resolution and/or a tracking accuracy of the real-time volumetric image data set may comprise and/or may be of the order of a fraction of a millimeter (mm) up to a few mm, e.g., of the order of 0.5 mm.

A temporal volatility may comprise an update rate, e.g., 30 frames per second for ultrasound, 5 to 10 updates per second for (e.g., X-ray) fluorescence imaging and/or an update every 10 milliseconds (ms) for a surgical needle and/or a catheter. Alternatively, or in addition, data from the surgical needle and/or catheter may have a higher temporal volatility than data from (e.g., X-ray) fluorescence imaging, which may have a higher temporal volatility than data from ultrasound.

The device may further comprise a rendering unit configured to render a real-time volumetric image of the part of the body based on the real-time volumetric image data set. Rendering the real-time volumetric image may also be denoted as displaying the real-time volumetric image. Alternatively, or in addition, the rendering unit may comprise a display.

The device may further comprise a storage unit configured to store the received data, any one of the iteratively combined data sets, and/or the real-time volumetric image data set and thus to store a final result and optionally intermediate results or a compressed representation thereof and/or of the computations.

The predefined criterion indicative of a temporal volatility may comprise a volatility index. Optionally, the volatility index may comprise a vanishing value for static data, a value of 1 for data updated for each real time image, and/or a value of 1/m for data updated after m real time images, wherein m comprises a natural number.

Alternatively, or in addition, a user input, e.g., by the medical practitioner before and/or during the interventional procedure, may comprise a volatility index larger than zero and at most 1, e.g., between 0.1 and 1.

The receiving unit may further be configured to periodically update the data from at least one or each of the multiple medical imaging sources. The combining unit may repeat the iterative combining of the q-th data with the (q-2)-th intermediary data responsive to the periodic update of the p-th data, wherein p ≤ q ≤ N+1.

The data from at least one medical imaging source with a temporal volatility higher than the lowest temporal volatility may be updated periodically. Alternatively, or in addition, the highest temporal volatility may be associated to the shortest updating periodicity associated to the medical imaging devices.

The step of iteratively combining the received data may be repeated starting from the first periodically updated data and/or the most recently updated data. E.g., if the third data is updated, the second intermediary data set may be updated using the updated third data set and the (e.g., unchanged) first intermediary data set.

The receiving unit is configured to receive the data from at least one or each of the multiple medical imaging sources in a first format and converting the data into a predetermined second format. The predetermined second format is universal for data received from any one of the multiple medical imaging sources. Alternatively, or in addition, the combining unit may be configured to combine the data from the multiple medical imaging sources in the predetermined second format.

The first format of data from multiple medical imaging sources may be based on at least one of a (e.g., semi-automatic and/or automatic) segmentation, a user editing from a (e.g., haptic) device, and/or a geometric function of a (e.g., tracked) device. E.g., an organ (e.g., comprising a liver and/or kidney) segmentation may be performed prior to the interventional procedure, e.g., using computed tomography (CT) and/or magnetic resonance imaging (MRI).

The first format may be specific to the at least one medical imaging source among the multiple medical imaging sources.

Alternatively, or in addition, the first format may differ among different medical imaging sources. Thus, the system is capable of processing a multi-format input.

Alternatively, or in addition, the data from at least one further or any one of the multiple medical imaging sources may be received at the receiving unit in the predetermined second format.

The second format from each of the multiple medical imaging sources may, e.g., comprise a signed distance field (SDF). The signed distance field (SDF) may also be denoted as signed distance function, or shortly as distance field. Alternatively, or in addition, the SDF comprises a position as an input and outputs a distance from the position to a nearest part of a shape (e.g., segmented organ and/or vessel and/or medical instrument and/or body or part thereof). Further alternatively, or in addition, the SDF comprises a subtraction of an inverted Euclidean distance transform (EDT) from an original EDT. Still further alternatively or in addition, a negative value of the output of the SDF may correspond to a position inside a contour of the shape and a positive value of the output of the SDF corresponds to a position outside the contour of the shape, or vice versa.

The contour and/or the shape may comprise a vessel wall and/or a (e.g., inner and/or outer) surface of an organ (e.g., liver and/or kidney).

The combined final data set may comprise a combined SDF.

The combining unit may further comprise at least one filter applied to the received data from at least one or each of the multiple medical imaging sources. Optionally, the at least one filter comprises an interpolation filter, an extrapolation filter, a three-dimensional Gaussian filter and/or a trilinear filter.

The at least one filter may remove predefined (also denoted as "unwanted") components and/or features from data received from at least one medical imaging source.

The interpolation filter may be configured to upsample data received from one or more medical imaging sources. Upsamling may comprise inserting zero-valued samples (e.g., zero-valued data) between original samples (e.g., between data acquired and/or obtained by a medical imaging source) to increase a sampling rate (also denoted as "zero-stuffing"). The interpolation filter may further be configured to filter the upsampled data. Upsampling and following filtering may alternatively be denoted as interpolating.

The interpolation filter may be configured to perform bilinear interpolations, e.g., by mapping a screen pixel location to a corresponding point on a texture map. A weighted average of attributes (e.g., transparency and/or color) of (e.g., four) surrounding texels may be computed and applied to the screen pixel. Bilinear interpolation comprises using (e.g., only) the four nearest pixels along diagonal directions (e.g., within a two-dimensional grid) from a given pixel to derive the weighted average of the attributes (e.g., transparency and/or color). The interpolation filter performing bilinear interpolations may also be denoted as and/or be comprised in a liner filter.

Alternatively, or in addition, the interpolation filter may perform trilinear interpolation, e.g., by mapping a voxel location to a corresponding point on a texture map. A weighted average of attributes (e.g., transparency and/or color) of (e.g., eight) surrounding texels may be computed and applied to the voxel, e.g. to a voxel value. Trilinear interpolation comprises using (e.g., only) the eight nearest voxels along diagonal directions (e.g., within a three-dimensional grid) from a given voxel to derive the weighted average of the attributes (e.g., transparency and/or color). The interpolation filter performing trilinear interpolations may also be denoted as and/or be comprised in a trilinear filter.

The extrapolation filter may be configured to extrapolate (e.g., synthetic) data at points in time in between acquisition times of data of one or more medical imaging sources. For example, for combining data of high temporal volatility (e.g., acquired and/or obtained from tracking a medical instrument, in particular a needle, used in the interventional procedure) with data of low temporal volatility (e.g., acquired and/or obtained from, in particular X-ray, fluorescence), the data of low temporal volatility may be extrapolated to the points in time when the data of high temporal volatility are updated.

The Gaussian filter may be configured to perform a convolution of acquired and/or obtained data with a Gaussian function (also denoted as performing a Weierstrass transform).

A size of the filter kernel may increase with a predetermined property of the received data. Optionally, the increase may be linear.

The filter kernel may also be denoted as convolution matrix and/or mask.

The predetermined property may comprise an indication of a distance, e.g., in terms of a SDF, comprised in the received data.

The (e.g., trilinear) filter and/or the (e.g., trilinear) interpolation may be implemented natively on a graphics processing unit (GPU) hardware. Mipmap (also denoted as MIP map or pyramid) is not conventionally a native GPU operation for three-dimensional (3D) textures. According to an embodiment implementing the iterative combining of the data from the multiple medical imaging sources (e.g., as a hierarchical 3D SDF reconstruction), the system, e.g., comprising the GPU, may determine the real-time volumetric image data set (e.g., reconstruct the SDF) at multiple resolutions and/or interpolate linearly between them (e.g., between the real-time volumetric image data set and/or the SDF as obtained using different resolutions).

The GPU may comprise an electronic circuit designed for rapidly manipulating and altering memory to accelerate the creation of images in a frame buffer intended for output to a display device.

The mipmap may comprise a pre-calculated optimized sequence of images, each of which is a progressively lower resolution representation of the previous. A width, height and/or depth of each image, or level, in the mipmap may be a factor of two smaller than the previous level.

Alternatively, or in addition, the combining unit may be configured for adaptive resolution and/or for including an adaptive region.

The adaptivity may relate to a spatial resolution and/or a temporal resolution (e.g., a frequency of updating) of data from one or more of the multiple medical image sources.

The real-time volumetric image data set may comprise and/or may be indicative of an ambient occlusion (AO) and/or a subsurface scattering (SSS) in relation to the part of the body based on the iteratively combined final data set.

An SSS ray direction may comprise the inverse of the SDF gradient.

AO may comprise a ratio of absorbed light rays to light rays emitted from any point cast towards a scene boundary (e.g., a hemisphere at infinite distance from the point, from which the light rays are emitted). By AO, objects such as vessel walls and/or organ surfaces may be detected as absorbing light rays.

Alternatively, or in addition, AO may comprise a shading and rendering technique used to calculate how exposed each point in a scene is to ambient light. Further alternatively, or in addition, AO may be indicative of an accessibility value determined at each surface point.

SSS may also be denoted as subsurface light transport (SSLT). SSS may result from light penetrating an object surface (the object comprising, e.g., the part of the body) and being scattered by inner material of the object (e.g., the part of the body) and exiting the surface at a different point, e.g., different from the point of a straight light ray.

The SSS may comprise a single scattering of the (e.g., light) ray. Alternatively, or in addition, the method may consider only a single direction of a ray. By considering only a single direction of a ray, a computational speed may be accelerated. The multiple medical imaging sources may be of heterogeneous type. The medical imaging sources being of heterogenous type may comprise, among at least two of the multiple medical imaging sources, a different classification in terms of latency, resolution, and/or accuracy.

The latency may also be denoted as updating frequency.

Data such as SDFs related to anatomical structures, e.g., obtained from CT and/or MRI, may be static and/or may comprise high resolution. Alternatively, or in addition, data such as SDFs derived from valves, catheters, wires, medical instruments and/or devices configured for insertion into and/or for contact with a patient's body may comprise a lower resolution than data such as SDFs related to anatomical structures. E.g., data such as SDFs derived from valves, catheters, wires, medical instruments and/or device may comprise a medium resolution and/or a low resolution.

Alternatively, or in addition, data such as SDFs derived from valves, catheters, wires, medical instruments and/or devices configured for insertion into and/or for contact with a patient's body may comprise real-time images.

The receiving unit may be configured to receive data from multiple medical imaging sources. The multiple medical imaging sources may comprise an (e.g., X-ray fluoroscopy device, an ultrasound device, a computed tomography (CT) device, a magnetic resonance imaging (MRI) device, and/or one or more medical instruments. The one or more medical instruments may comprise a catheter, a stent, a balloon, a wire and/or a valve.

The medical instruments may be configured for guiding through human pathways, e.g., blood vessels, and/or for percutaneous guidance.

The data from at least one of the multiple medical imaging sources, e.g., from a CT device and/or an MRI device, may be obtained and/or acquired before the data from at least one further medical imaging source. E.g., the data from the at least one medical imaging source, e.g., from the CT device and/or the MRI device, may be stored (also: "pre-stored") in the device and/or processed when data from the at least one further medical imaging source become available, e.g., in real-time and/or during the interventional procedure.

Alternatively, or in addition, the data from at least one medical imaging source (e.g., the data from the at least one further medical imaging source) may be obtained and/or acquired (e.g., approximately) in real-time, e.g., from ultrasound and/or (e.g., X-ray) fluoroscopy.

The device may comprise a graphics processing unit (GPU). Optionally, at least the step of iteratively combining the received data may be performed by the GPU.

Alternatively, or in addition, the device may comprise, or may be comprised in, a one or more workstations and/or a computing cluster.

At least the step of iteratively combining the data may be performed by a dedicated GPU software, e.g., OpenGL compute shader.

The outputting unit and/or the rendering unit may comprise a transfer function configured for outputting the real-time volumetric image data set and/or for rendering the volumetric image according to a predefined distance imaging scale. Optionally, the predefined distance imaging scale may comprise a color scale and/or a gray shades scale.

The transfer function may comprise an optical transfer function (OTF) and/or a modulation transfer function (MTF). Alternatively, or in addition, the transfer function may specify how different optical values are handled by a system.

A value from a medical imaging source (also briefly denoted as scanner value), e.g., comprising a value on a Hounsfield scale (also denoted as Hounsfield Unit, HU), may be mapped to an optical value for use by the outputting unit and/or the rendering unit, e.g., by means of a transfer function. E.g., HU may be applied to CT images. For example, bone structure may comprise HU values of 300 to 1900, and soft tissue may comprise HU values from 100 to 300. Alternatively or in addition, the optical value may comprise an optical opacity (e.g., a value alpha). Further alternatively or in addition, a one-dimensional transfer function may map a HU to an optical value, e.g., comprising an optical color and/or opacity.

By the transfer function, scanner values may be mapped to visible and/or optical properties, in particular to color and/or transparency (and/or opacity) of a volumetric image.

Up to now, the invention has been described with respect to the claimed apparatus or device. Features, advantages or alternative embodiments mentioned with respect to the apparatus can also be assigned, transferred or, applied to the other claimed or described subject matters (e.g. method, the computer program or a computer program product) and vice versa. In other words, the subject matter of the apparatus/system can be improved or further developed with features described or claimed in the context of the method and vice versa. In this case, the functional features of the method are embodied by structural units of the system, configured to dedicatedly execute this function and vice versa, respectively. Generally, in computer science at least from a computational point of view, a software implementation and a corresponding hardware implementation are equivalent, at least from a computability perspective. Thus, for example, a method step for "storing" data may be performed with a "storage unit" and respective instructions to write data into the storage. For avoiding redundancy, these embodiments are not reiterated or explicitly described again for the apparatus, because they have been described already in relation to the method.

Wherever not already described explicitly, individual embodiments, or their individual aspects and features, described herein can be combined or exchanged with one another, even when mentioned in other embodiments, without limiting or widening the scope of the described invention, whenever such a combination or exchange is meaningful and in the sense of this invention. Accordingly, any feature may be combined with at least one other feature, which is claimed and/or described in this application.

As to a second aspect, a computer-implemented method for real-time volumetric imaging from multiple medical imaging sources of a part of a body during an interventional procedure according to claim 14 is provided.

The order, according to which the steps of the method of the present invention are described in the present specification, does not necessarily reflect the chronological order, according to which said steps are carried out.

The method may further comprise one or more steps for real-time volumetric imaging from multiple medical imaging sources of a part of a body during an interventional procedure using the device according to or corresponding to any feature disclosed in the context of the first and/or the device aspect.

As to a third aspect, a computer program product according to claim 15 is provided.

Optionally, the computer program product may be stored on a computer-readable medium. The computer program product may also be provided for download, e.g., via a radio or cellular network, the Internet and/or a host computer. Alternatively, or in addition, the method may be encoded in a Field-Programmable Gate Array (FPGA) and/or an Application-Specific Integrated Circuit (ASIC), or the functionality may be provided for download by means of a hardware description language.

### BRIEF DESCRIPTION OF DRAWINGS

Further details of embodiments of the technique are described with reference to the enclosed drawings, wherein:
- Fig. 1: shows an example schematic block diagram of a device for real-time volumetric imaging from multiple medical imaging sources of a part of a body during an interventional procedure;
- Fig. 2: shows an exemplary embodiment of a computing device comprising a graphics processing unit (GPU), which may embody the device of Fig. 1;
- Fig. 3: shows an example flowchart of a method of real-time volumetric imaging from multiple medical imaging sources of a part of a body during an interventional procedure, which method may be implementable by the device of Fig. 1;
- Fig. 4: shows an example of path tracing multiple light rays from a point towards a scene boundary for determining ambient occlusion (AO);
- Fig. 5: shows two examples of a signed distance function (SDF) for two points P and Q at a fixed distance d, on the left hand side without occlusion object and on the right hand side with occlusion object; and
- Fig. 6: shows an example set-up for determining a distance of light traveled through an object using a depth map for subsurface scattering (SSS).

### DETAILED DESCRIPTION

In the following description, for purposes of explanation and not limitation, specific details are set forth, such as a computing device in order to provide a thorough understanding of the technique disclosed herein. It will be apparent to one skilled in the art that the technique may be practiced in other embodiments that depart from these specific details.

Moreover, those skilled in the art will appreciate that the functions, steps, units and modules explained herein may be implemented using software functioning in conjunction with a programmed microprocessor, an Application Specific Integrated Circuit (ASIC), a Field Programmable Gate Array (FPGA), a Digital Signal Processor (DSP) or a general purpose computer, e.g., including an Advanced RISC Machine (ARM). It will also be appreciated that, while the following embodiments are primarily described in context with methods and devices, the invention may also be embodied in a computer program product as well as in a system comprising at least one computer processor and memory coupled to the at least one processor, wherein the memory is encoded with one or more programs that may perform the functions and steps or implement the units and modules disclosed herein.

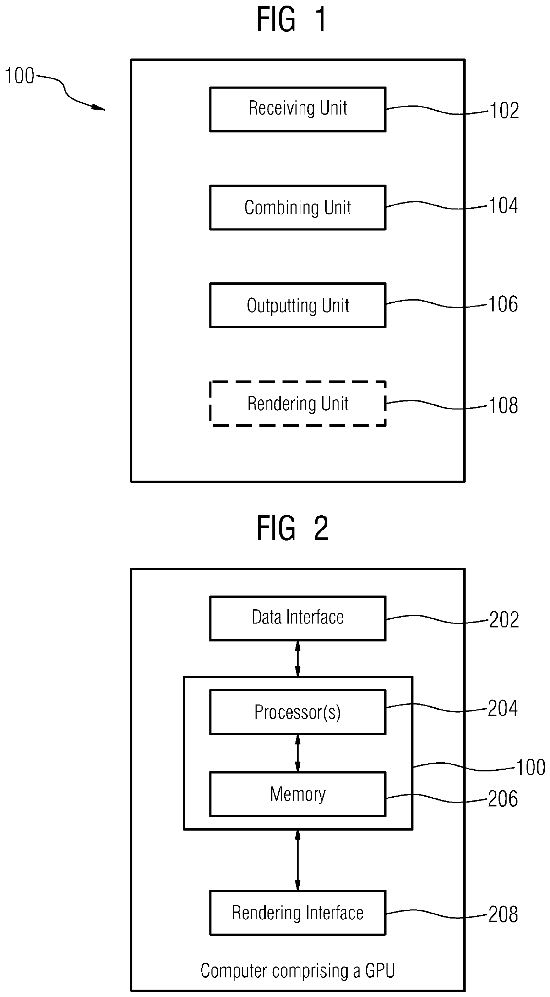

Fig. 1 schematically illustrates an example block diagram of a device for real-time volumetric imaging from multiple medical imaging sources of a part of a body during an interventional procedure. The multiple medical imaging sources comprise N+1 medical imaging sources of the part of the body with N greater or equal to two. The device is generally referred to by reference sign 100.

The device 100 comprises a receiving unit 102 configured to receive data regarding the part of the body from each of the multiple medical imaging sources.

The device 100 further comprises a combining unit 104 configured to iteratively combine the received data from each of the multiple medical imaging sources.

Iteratively combining the data comprises ordering the data according to a predefined criterion indicative of a temporal volatility of the data based on the medical imaging source among the multiple medical imaging sources from which the data was received.

Iteratively combining the data further comprises combining the data of the two medical imaging sources among the multiple medical imaging sources with the two lowest temporal volatilities into a first intermediary combined data set.

Iteratively combining the data still further comprises combining the first intermediary data set and the data from a third medical imaging source among the multiple medical imaging sources with the third lowest temporal volatility into a second intermediary combined data set.

Iteratively combining the data still further comprises optionally combining the second intermediary data set (e.g., for N greater or equal to three) and/or any further intermediary combined data set with the data from a fourth (e.g., for N greater or equal to four) and/or any further (e.g., for N greater than four) medical imaging source with the next-lowest temporal volatility into a still further intermediary combined data set.

Iteratively combining the data still further comprises identifying the N-th (and/or the last possible) combined data set of the iteration as a combined final data set.

The device 100 still further comprises an outputting unit 106 configured to output a real-time volumetric image data set regarding the part of the body based on the iteratively combined final data set.

Optionally, the device 100 further comprises a rendering unit 108 configured to render a real-time volumetric image of the part of the body based on the real-time volumetric image data set.

Any of the units of the device 100 may be implemented by modules configured to provide the corresponding functionality. Fig. 200 shows a schematic block diagram for an embodiment of the device 100. The device 100 comprises processing circuitry, e.g., one or more processors 204 for performing the method 300 (further detailed below) and memory 206 coupled to the processors 204. For example, the memory 206 may be encoded with instructions that implement at least one of the units 102, 104, 106 and 108.

The one or more processors 204 may be a combination of one or more of a microprocessor, controller, microcontroller, central processing unit, digital signal processor, application specific integrated circuit, field programmable gate array, or any other suitable computing device, resource, or combination of hardware, microcode and/or encoded logic operable to provide, either alone or in conjunction with other components of the device 100, such as the memory 206, transmitter functionality. For example, the one or more processors 204 may execute instructions stored in the memory 206. Such functionality may include providing various features and steps discussed herein, including any of the benefits disclosed herein. The expression "the device being operative to perform an action" may denote the device 100 being configured to perform the action.

As schematically illustrated in Fig. 2, the device 100 may be embodied by a computer, in particular a workstation, comprising a GPU.

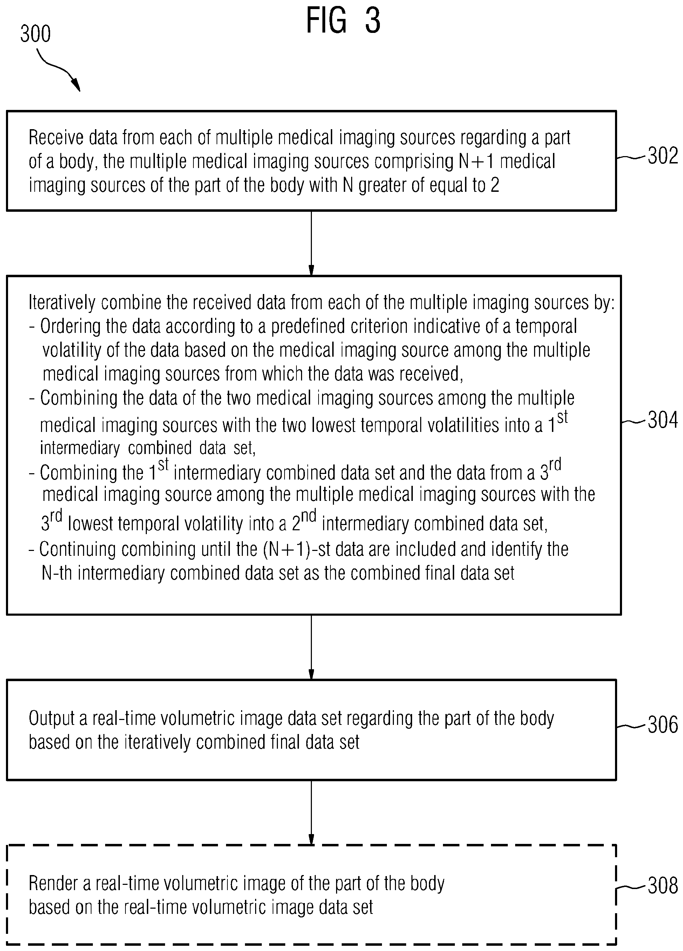

Fig. 3 schematically illustrates an example flowchart for a method 300 for real-time volumetric imaging from multiple medical imaging sources of a part of a body during an interventional procedure. The multiple medical imaging sources comprise N+1 medical imaging sources of the part of the body with N greater or equal to two.

The method 300 comprises or initiates a step 302 of receiving data from each of the multiple medical imaging sources regarding the part of the body.

The method 300 further comprises or initiates a step 304 of iteratively combining the received data from each of the multiple medical imaging sources.

Iteratively combining the data comprises ordering the data according to a predefined criterion indicative of a temporal volatility of the data based on the medical imaging source among the multiple medical imaging sources from which the data was received. Iteratively combining the data further comprises combining the data of the two medical imaging sources among the multiple medical imaging sources with the two lowest temporal volatilities into a first intermediary combined data set. Iteratively combining the data further comprises combining the first intermediary data set and the data from a third medical imaging source among the multiple medical imaging sources with the third lowest temporal volatility into a second intermediary combined data set.

Iteratively combining the data still further comprises, e.g., for N greater or equal to three, combining the second intermediary data set and/or, e.g., for N greater or equal to four, the third intermediary combined data set with the data from a fourth medical imaging source and/or any further medical imaging source with the next-lowest temporal volatility into a still further intermediary combined data set.

Iteratively combining the data still further comprises identifying the N-th (e.g., the last possible) combined data set of the iteration as a combined final data set.

The method 300 further comprises or initiates a step 306 of outputting a volumetric image data set regarding the part of the body based on the iteratively combined final data set.

Optionally, the method 300 comprises a step 308 of rendering a real-time volumetric image of the part of the body based on the real-time volumetric image data set.

The method 300 may be performed by the device 100. For example, the units 102, 104, 106 and 108 may perform the steps 302, 304, 306 and 308, respectively.

By the method 300, a novel approach for combining data (in particular SDFs) based on data from multiple, in particular heterogenous, medical imaging sources of a part of a body is provided that allows to obtain a combined real-time volumetric image data set suitable for use during an interventional procedure.

According to a first embodiment, the data to be combined comprises SDFs of a three-dimensional (3D) volume.

According to a second embodiment, which is combinable with any other embodiment disclosed herein, the real-time volumetric image data set comprises an ambient occlusion (AO) and/or subsurface scattering (SSS) effects that greatly reduce the rendering latency for volume rendering to support their use in a real-time procedure, in particular during interventional procedures.

According to a third embodiment, which is combinable with any other embodiment disclosed herein, the medical imaging sources comprise heterogeneous sources such as automatic segmentation, user editing from haptic devices, and/or geometric functions of tracked devices. The various sources of the data, e.g., SDFs, are generated and updated using different techniques with different latency, different resolution, and/or different accuracy. By the device and the method disclosed herein, the data from various sources, e.g., the SDFs, can be combined into a single combined SDF in real time, in particular on a GPU.

By the method 300, sampling and rendering computations of multiple medical imaging sources (also denoted as "input sources") is reduced in an interventional procedure comprising a rendering scene by first creating a combined final data set (or briefly "combined data set"), e.g., a combined SDF, stage before the rendering and sampling step of the computational pipeline.

Structures extracted from a 3D planning volume such as organs, lesions, vasculatures, and/or manual markings and roadmap landmarks, together with real-time imaging and/or medical instruments are first combined into a single combined final data set, e.g., a combined SDF volume. The single combined final data set, e.g., the combined SDF volume, can be used during the render stage, e.g., in real-time during an interventional procedure, to apply, e.g., ambient occlusion and subsurface scattering, effects with a stable rendering performance.

Some of the multiple medical imaging source(s) may initially provide its (or their) individual data in a common and/or second format, e.g., their own SDF. Some data from other medical imaging sources among the multiple medical imaging sources may initially comprise data in a first format that are converted into the second format, e.g., SDFs, on-the-fly. For example, segmentation of anatomical structures (e.g., comprising an organ such as a liver and/or kidney) from 3D CT might be carefully segmented using an automatic or semi-automatic method and output as a SDF representing a distance to the boundary of the segmented anatomical structure (e.g., comprising the organ such as the liver and/or the kidney).

A catheter tip, wire, and/or valve (as examples of medical instruments and/or devices as one medical imaging source) may be tracked on a live tow-dimensional (2D) fluoroscopy image using pose estimation from the real-time image based on an a priori model of the medical instrument and/or device.

The 3D location of the medical instrument and/or device can be derived by combining the tracked position in the 2D image and the known projection field of view of data from further medical imaging sources, e.g., comprising one or more angiography data (e.g., X-ray fluorescence images and/or MRI data) from a C-Arm.

Subsequently the 3D location can be computed iteratively as values of intermediary data sets, e.g. SDFs, by computing the distance to the tracked position and/or "procedurally" (during the interventional procedure).

An SDF may be obtained by determining (e.g., computing) a distance from every point comprised in an SDF grid to the closest point on a device surface. **E.g.,** a computer-aided design (CAD) model of the device may comprise a surface mesh. The position and/or orientation of the device may be determined **(e.g.,** detected) in 3D space. The position and/or orientation may be sufficient for determining the closest distance from any 3D point. Alternatively or in addition, by this an explicit SDF is determined.

Alternatively or in addition, an implicit SDF and/or a procedurally defined SDF may be determined. The distance values need not be pre-determined (e.g., determined before the interventional procedure and/or pre-computed) and/or stored, but may be determined (e.g., computed) as needed. E.g., when shading a (e.g., predetermined and/or current) location during rendering, the distance to the tracked device may be determined (e.g., computed) for the (e.g., predetermined and/or current) shading location only. Alternatively or in addition, as an optimization, the determining (e.g., the computation and/or calculation) may comprise determining the distance to the tracked point, e.g., rather than than to the surface of the device.

According to an embodiment, the explicit SDG is determined (e.g., computed) and stored for the surface of the device, e.g., in order to allow for accurate proximity determinations (e.g., calculations).

For rigid devices, a fixed SDF may be pre-determined (e.g., determined before the interventional procedure and/or pre-computed) based, e.g., on a CAD model. The SDF may be sampled and/or resampled based on the detected position and/or orientation (e.g., of the part of the human body and/or of a non-rigid device, in particular a medical instrument used during the interventional procedure) when combining SDFs. Alternatively or in addition, during the shading of a (e.g., predetermined and/or current) point using the outputting unit and/or the rendering unit, the (e.g., predetermined and/or current) location may be transformed to the coordinate system of the fixed location SDF using the (e.g., predetermined and/or current) position and/or orientation. This embodiment exemplifies procedural sampling.

For some medical instruments and/or devices, tracking hardware may track the position and/or orientation of the medical instrument and/or device. The prior geometry knowledge may be used to calculate the data, e.g., SDF, associated to the medical instrument and/or device. Alternatively or in addition the device (e.g., comprising a medical instrument) position and/or orientation may be tracked using hardware trackers (e.g., a gyroscope, inertial tracker and/or optical tracking), e.g., as opposed to pose estimation from imaging. The outcome (e.g., result) of any step and/or tracking method may comprise a position and/or orientation for one or more locations of the device and/or medical instrument.

Due to the heterogeneous medical imaging sources of the part of the body (e.g., viewed as different objects), the data, e.g., the SDFs, are also heterogeneous in terms of the resolution, accuracy, and/or updating frequency. Anatomical structure data, e.g., anatomical structure SDFs, comprise conventionally static data and/or high-resolution data. Medical instrument (e.g., valves, catheter and/or other devices) data from a real-time medical imaging source used during the interventional procedure may comprise a different resolution due to the imaging modality properties and (e.g., real-time) computation limitation. At the same time, the medical instrument data are often updated in high frequency.

To determine AO and SSS effects, multiple SDFs are combined into one combined SDF in real time. Given a set of (e.g., individual) SDFs, each SDF within the set may be classified into one of two sets: static and dynamic.

Static SDFs do not change during the interventional procedure. Static SDFs can be precomputed and combined into one static SDF, e.g., as the first combined data set in case of two static SDFs.

Dynamic SDFs are obtained (e.g., computed) from real-time imaging. Due to the complexity of the algorithm used to determine each SDF, some may be updated in real time (e.g., valves in ultrasound). Others may be updated in several frames (e.g., catheter tip and/or wire). Each kind of dynamic SDF is one input of the combining algorithm.

To each data obtained from a medical imaging device, e.g., an SDF, a (e.g., normalized) volatility index may be assigned. The (E.g., normalized) volatility index may be determined by the time spent to update once divided by the time to capture one real-time image.

Static data (e.g., a static SDF) may have volatility index 0.

For dynamic data (e.g., a dynamic SDF) is updated for each real time image, the (e.g., normalized) volatility index may be 1.

For dynamic data (e.g., a dynamic SDF) that is updated for every n real time images, the (e.g., normalized) volatility index may be 1/*n*. Alternatively, or in addition, the (e.g., normalized) volatility index may be updated progressively (e.g., for every real time image, 1/*n* part of the data, in particular the SDF, is updated and made available). Then the volatility index is still 1.

User input (e.g., by a practitioner) may have a predefined low (e.g., normalized) volatility index, e.g., 0.1. When the user input comes in, the volatility index may be increased (e.g., up to 1), e.g., since the user input might come in soon. If and/or after the user input come, the volatility index may decrease (e.g., down to 0.1).

The combining algorithm may periodically check which data (e.g., which SDF) has been updated before providing the real-time volumetric image data set for rendering a fused image. If so, the combining algorithm may combine the updated data, e.g., the updated SDF, with other data, e.g., other SDFs.

The combining algorithm may order (also: sort) the data, e.g., the SDFs, with ascending volatility indices in a sequence **{*d*0*,*d*1,*d*2*,.*.. ,*d*n}.*** E.g., each SDF comprises a 3D volume that may be uploaded to the device, e.g., comprising a GPU, as a 3D texture. The combining algorithm may combine the first data d0 with the second data d1 and store the result, e.g., the first intermediary combined data set, *c*1 in a 3D texture. The combining algorithm to combine two pieces of data, e.g., two SDFs, may be executed on a GPU with a dedicated software, e.g., OpenGL compute shader. The compute shader can combine two 3D textures in one pass efficiently.

The combining algorithm may continue to combine the first intermediary combined data set *c*1 with the third data d2 and store the result in the second intermediary combined data set *c2.*

The combining algorithm may continue until the last (e.g., intermediary) combined data set, cn, is determined as the combined final data setl, e.g., the combined SDF, used for rendering.

Interpolation and extrapolation filters may be used to combine data from medical imaging sources with different streaming rates and latencies. E.g., predictive extrapolation may be used with high latency (e.g., SDF) medical imaging sources, and/or a high-order interpolation filter may be used with a low-rate (e.g., SDF) medical imaging source.

Depending on performance constraints, adaptive resolution, adaptive region and/or hierarchical reconstruction methods may be used when determining the combined final data set, e.g., the combined SDF. In the combining process, *n* - 1 intermediary data sets, e.g., intermediary SDFs, are determined, e.g., in addition to the combined final data set, e.g., the combined SDF. Depending on (e.g., GPU) memory limitations, all or some of the intermediary data sets may be stored as cached results. The next time when data, e.g., an SDF, from a medical imaging source is updated, the algorithm may check the position of the updated date in the ordered sequence of data (e.g., the sorted SDF sequence) and starts the update process from an available cached result (e.g., a cached intermediary combined data set, in particular a cached intermediary combined SDF) before the updated data to reduce the computation time. Since the data (e.g., SDFs) are ordered based on the volatility index, previous cached results (e.g., previous intermediary combined data sets) are available most of the time during the processing of dynamic data, e.g., dynamic SDFs, from real-time medical imaging sources.

With the combined SDF, ambient occlusion (AO) may be determined at the rendering stage. In contrast to the conventional rendering of meshes with AO effect as described in "Advanced Real-Time Rendering in 3D Graphics and Games, Chapter 9 Fast Approximations for Global Illumination on Dynamic Scenes", SIGGRAPH 2006, embodiments of the invention provide a technique for rendering volumes with AO effect.

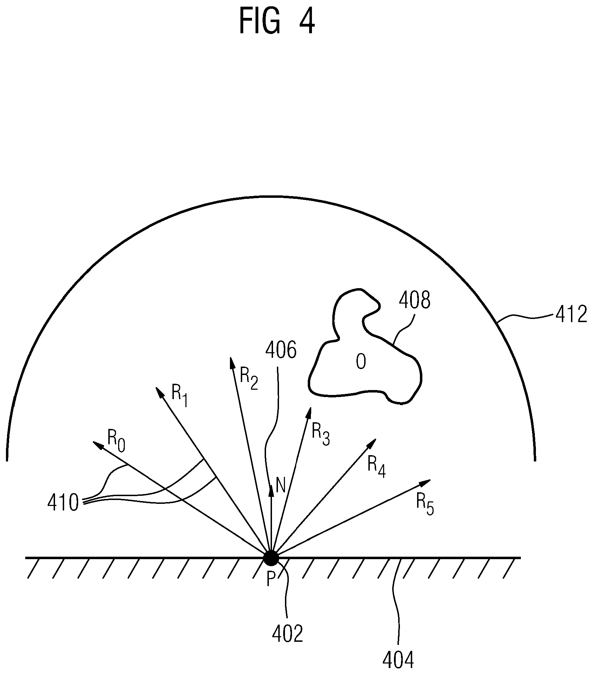

Fig. 4 depicts an example of an AO effect, which may be evaluated with Monte-Carlo ray tracing techniques. At each point P, as exemplified at reference sign 402 lying on a surface 404 with normal direction N at reference sign 406, to be shaded, a large number of rays (exemplified by R0, R1, R2, R3, R4 and R5) at reference sign 410 are cast towards the scene boundary 412, e.g., considered to be an evenly light emitting hemisphere centered at the origin of the scene and of infinite radius. These rays 410 bounce off from objects 408 in the scene, until they are eventually considered to be absorbed or reach the boundary 412. The ratio of ray absorption is the AO term. Conventionally determining the AO from the large number of rays at reference sign 410 is computationally intensive and time-consuming and/or quite slow due to the large number of rays 410 traced at every sampling point 402.

In "Advanced Real-Time Rendering in 3D Graphics and Games, Chapter 9 Fast Approximations for Global Illumination on Dynamic Scenes", SIGGRAPH 2006, an SDF was employed to approximate a surface curvature in meshes.

Embodiments of the invention allow for approximating a curvature of the volumetric objects, in particular vessel walls and/or surfaces of organs such as liver and/or kidney, by one or more SDFs. The curvature information may be used to darken a volume wherever it has significant 'concavity', which approximates an AO effect.

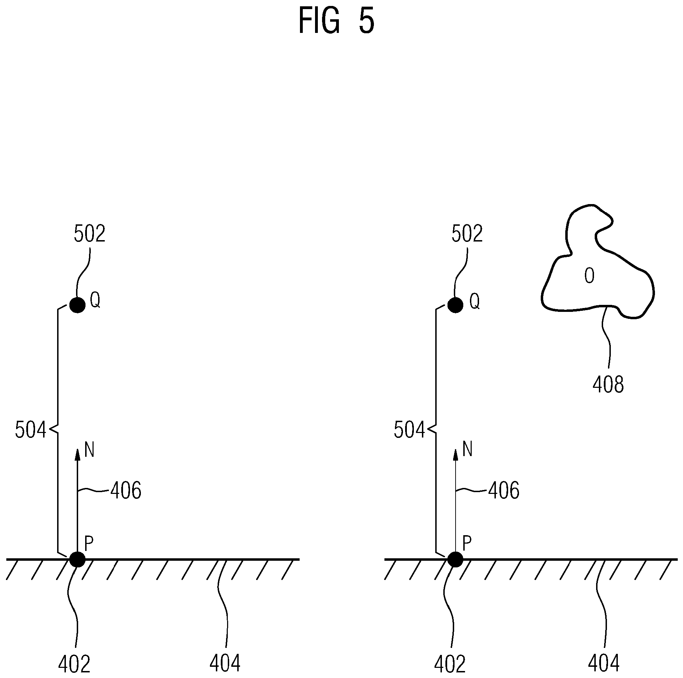

Fig. 5 depicts an example of objects 408, e.g., vessel walls and/or a surface of an organ such as liver and/or kidney, at a larger distance having a lesser, 'blurrier' AO effect than those close to the point P at reference sign 402. A light ray (also denoted as AO ray) is shot from sampling point P at reference sign 402 on a (e.g., object) surface 404 along the normal direction N at reference sign 406.

For any point Q=P+dN, at reference sign 502 with distance d at reference sign 504 between P and Q, on the AO ray, if there is no occlusion object 408 present, the sampled SDF value at Q is -d. If there is an occlusion object 408 present, the light is attenuated by the factor SDF(P+dN)+d.

The total light attenuation of an AO ray is determined by multiplying the attenuation factors on all sampling points on the AO ray. To simulate the blurry effect, the SDF is sampled with a filter, e.g., a 3D Gaussian filter or trilinear filter. The filter kernel size may be selected proportionally to the distance d. For efficiency on current hardware, e.g., a trilinear filter may be efficiently implemented with mipmap texture on a GPU to achieve various kernel sizes with mipmap levels.

For rendering volumes with AO effect, a ray tracing algorithm is used. For every pixel on the rendered image, a primary ray is shot to the scene. The ray starts at the image pixel, and the direction of the ray is defined by the image projection direction. For every sampling point on the primary ray, both the volume and the SDF are sampled.

If the sampled distance value from the SDF is, e.g., less than 0, then the sampling point is classified as outside the tissue, vessel or organ, e.g., outside a vessel wall or an organ such as liver and/or kidney. A transfer function is used to color the sampling point, similar to conventional ray casting algorithms. If the sampled distance value is 0 (e.g., on the object surface) or, e.g., greater than 0, then sampling point P is inside the organ, vessel or tissue, e.g., a vessel wall or an organ such as liver and/or kidney. The lighting at this sampling point is attenuated for AO and SSS effects.

A SSS effect results from light penetrating object surfaces and being scattered by inner material, which is quite common in semitransparent human skin and tissues. An inherent property of semitransparent materials is absorption. The further through the material light travels, the more light is absorbed. Another obvious property is a general blurring of the diffuse lighting. Monte-Carlo ray tracing may be used to determine the SSS effect, but it is computationally expensive and slow.

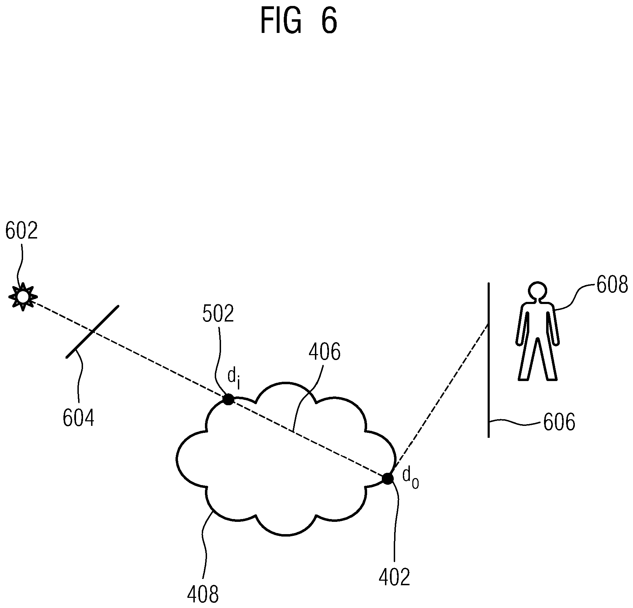

A real-time method called depth map based SSS is applied in "Real-time Approximations to Subsurface Scattering", GPU Gems, Addison-Wesley Professional: 263-278, 2004, to estimate a distance that light travels from a surface through the material to the inner point. It renders the scene from the point of view of the light source 602 in Fig. 6 to a depth map and stores the distance to the nearest surface. When shading a point, the distance from this point (d0 at reference sign 402 in Fig. 6) to the light source 602 is subtracted with the distance from the light source 602 to the nearest surface (di) at reference sign 502, which is the estimated distance the light travels through the object 406. The conventional depth map assumes that the material inside the object 406 is uniform and a point light source 602 or directional light source 602 is used. The conventional depth map does not consider diffusion of light when passing through the object 406. Diffusion, however, usually occurs when considering (e.g., a part of a) human body as the object 406.

Embodimtents of the invention may use ambient light. Moreover, an organ and/or tissue intensity may vary under the boundary.

According to the technique presented herein, an SSS ray may be cast from a sampling point P 402 along a direction N 406, as exemplified in Figs. 4 and 5. The SSS ray direction N 406 may comprise, or may correspond to, the inverse of the SDF gradient, e.g., instead of the anatomy volume gradient. The volume gradient inside an anatomy structure might be insignificant, or even incorrect, due to noise, while the SDF is always smooth and accurate.

The intersection point R of the SSS ray on the object surface is the nearest surface point to sampling point P, e.g., as depicted at reference sign 402 for P and/or 502 for R. To determine the light absorption from the nearest surface point to the sample point, e.g., at reference sign 402 for P and/or 502 for R, the technique according to the invention samples the anatomy volume and uses the transfer function to get an optical opacity (e.g., alpha), similarly to conventional ray casting algorithms. E.g., a 3D Gaussian filter, which may be applied for example for better quality, and/or trilinear filter, which may be applied for example for higher speed, may be used.

To estimate light diffusion, a filter kernel size may increase while marching the ray from P to R. The closer the sampling point is to the surface, the larger filter kernel size may be. At surface point R, the AO ray is cast to determine the incoming light for AO effects as described herein.

Fig. 6 shows an example of a depth map 604, an image plane 606 and a user (also denoted as "viewer") 608 of the volumetric image data set and/or the medical instrument during an interventional procedure in relation to the light source 602, the object 408 (e.g., a vessel and/or organ such as liver and/or kidney), light ray (also denoted as AO ray) 406 and the points R and P at reference signs 502 and 402, respectively.

The inventive technique (e.g., only) casts one ray at every sampling point inside the object (e.g., with sign choice SDF>0 and/or a positive value of the SDF). The ray may be scattered (e.g., only) once at the object surface (SDF=0 and/or a vanishing value of the SDF).

The computational speed is similar to the conventional shadow ray method, which casts a shadow ray towards the light source at every sampling point. Thus, the technique provides real-time volumetric imaging, e.g., on modern GPUs. By contrast, the conventional Monte-Carlo ray tracing cannot be interactive on modern computing devices, e.g., comprising the latest generation of GPU.

By the inventive technique, a global AO effect is taken into account and/or captured. By contrast, conventional interactive volumetric AO methods, as described by Ropinski et al. in "Interactive Volume Rendering with Dynamic Ambient Occlusion and Color Bleeding", Comput. Graph. Forum. 27., 567-576 (2008*),* and by Kroes et al. in "Smooth Probabilistic Ambient Occlusion for Volume Rendering" (2018), only consider local histograms or neighborhoods for local occlusions.

According to an exemplary embodiment of the invention, the data from multiple medical imaging sources comprise at least one SDF per medical imaging device. At least three different medical imaging sources differ in their update rates and/or the temporal volatility of their data.

(*d*₀) denotes the first SDF from organ segmentation on preoperative data and/or data acquired before the interventional procedure, e.g., CT or MR scans, having high precision and resolution, and comprising static and/or the lowest temporal volatility.

(*d*₁) denotes the second SDF from device tracking on live imaging data, e.g., ultrasound or DynaCT data during the interventional procedure having medium precision and/or resolution and a medium temporal volatility.

(*d*₂) denotes the third SDF from device tracking with sensors attached to the device, e.g., optical, magnetic and/or inertial tracking, in particular of a medical instrument used in the interventional procedure. Sensor fusion may also be used. The data (*d*₂) has very high precision, a very high update rate and a very high temporal volatility.

During the interventional procedure, the combined SDF for the static (e.g., the first) and the slow update (e.g., the second) medical imaging sources is determined as *c*₁ = *d*₀ + *d*₁, where the first intermediary combined SDF *c*₁ stores the cached intermediate result and is updated at the higher rate of the two medical imaging sources, namely at the update rate of ***d*₁**.

In a following step, the final SDF (also denoted as combined final SDF, or shortly combined SDF) is computed as *c*₂ ***=** c*₁ *+ d*₂, and the update rate matches the high acquisition rate of the *d*₂ data source.

In a further embodiment, the data *d*₁ may be temporally upsampled to match the update rate of *d*₂ before the combined SDFs are computed, **e.g.,** by using extrapolation techniques.

By the device, method and computer program product as disclosed herein, a novel technique for combining data obtained and/or acquired from medical imaging devices, in particular signed distances fields (SDFs) of 3D volumes, for significantly reducing a rendering latency for enabling real-time volumetric imaging during interventional procedure, **e.g.,** on a human body, is provided. Exemplary embodiments apply SDFs for determining ambient occlusion (AO) and subsurface scattering (SSS) effects for the real-time volumetric imaging.

Embodiments of the real-time volumetric imaging technique have been described in the context of an interventional procedure performed on a human body. The technique is, however, equally applicable to interventional procedures performed on animals.

Many advantages of the present invention will be fully understood from the foregoing description, and it will be apparent that various changes may be made in the form, construction and arrangement of **(e.g.,** the sub-networks of) the neural network system and the associated method without departing from the scope of the invention and/or without sacrificing all of its advantages. Since the invention can be varied in many ways, it will be recognized that the invention should be limited only by the scope of the following claims.

### List of Reference Signs

| | |
|---|---|
| Device | 100 |
| Receiving Unit | 102 |
| Combining Unit | 104 |
| Outputting Unit | 106 |
| Rendering Unit | 108 |
| Data Interface | 202 |
| Processor(s) | 204 |
| Memory | 206 |
| Rendering Interface | 208 |
| Step of receiving data | 302 |
| Step of iteratively combining data | 304 |
| Step of outputting a real-time volumetric data set | 306 |
| Step of rendering a real-time volumetric image | 308 |
| Sampling Point P | 402 |
| Object Surface Comprising Sampling Point P | 404 |
| Normal direction | 406 |
| Occlusion Object | 408 |
| Ray | 410 |
| Scene boundary | 412 |
| Point Q | 502 |
| Distance d | 504 |
| Light Source | 602 |
| Depth Map | 604 |
| Image Plane | 606 |
| Observer | 608 |

### LIST OF CITED STATE OF THE ART (in order of appearance)

[1] Advanced Real-Time Rendering in 3D Graphics and Games, Chapter 9 Fast Approximations for Global Illumination on Dynamic Scenes, SIGGRAPH 2006;
[2] Real-time Approximations to Subsurface Scattering, GPU Gems, Addison-Wesley Professional: 263-278, 2004;
[3] Ropinski, Timo & Meyer-Spradow, Jennis & Diepenbrock, Stefan & Mensmann, Jörg & Hinrichs, Klaus. (2008). Interactive Volume Rendering with Dynamic Ambient Occlusion and Color Bleeding. Comput. Graph. Forum. 27. 567-576;
[4] Kroes, Thomas & Schut, Dirk & Eisemann, Elmar. (2018). Smooth Probabilistic Ambient Occlusion for Volume Rendering.

## Claims

1. A device (100) for real-time volumetric imaging from multiple medical imaging sources of a part of a body during an interventional procedure, the multiple medical imaging sources comprising N+1 medical imaging sources of the part of the body with N greater or equal to two, the device (100) comprising:
- a receiving unit (102) configured to receive data regarding the part of the body from each of the multiple medical imaging sources, wherein the receiving unit (102) is configured to receive the data from at least one or each of the multiple medical imaging sources in a first format and converting the data into a predetermined second format, wherein the predetermined second format is universal for data received from any one of the multiple medical imaging sources;
- a combining unit (104) configured to iteratively combine the received data from each of the multiple medical imaging sources, wherein the combining unit (104) is configured to combine the data from the multiple medical imaging sources in the predetermined second format, and wherein iteratively combining the data comprises
- ordering the data according to a predefined criterion indicative of a temporal volatility of the data based on the medical imaging source among the multiple medical imaging sources from which the data was received,
- combining the data of the two medical imaging sources among the multiple medical imaging sources with the two lowest temporal volatilities into a first intermediary combined data set,
- combining the first intermediary data set and the data from a third medical imaging source among the multiple medical imaging sources with the third lowest temporal volatility into a second intermediary combined data set, and
- in case of N greater or equal to three, combining the second intermediary data set and/or any further intermediary combined data set with the data from a fourth and/or any further medical imaging source with the next-lowest temporal volatility into a still further intermediary combined data set, and
- identifying the N-th combined data set of the iteration as a combined final data set; and
- an outputting unit (106) configured to output a real-time volumetric image data set regarding the part of the body based on the iteratively combined final data set;
wherein the receiving unit (102) is further configured to periodically update the data from at least one or each of the multiple medical imaging sources, and wherein the combining unit repeats the iterative combining of the q-th data with the (q-2)-th intermediary data responsive to the periodic update of the p-th data, wherein p ≤ q ≤ N+1.

2. The device (100) of claim 1, further comprising a rendering unit (108) configured to render a real-time volumetric image of the part of the body based on the real-time volumetric image data set.

3. The device (100) of claim 1 or 2, further comprising a storage unit (206) configured to store the received data, any one of the iteratively combined data sets, and/or the real-time volumetric image data set.

4. The device (100) of any one of claims 1 to 3, wherein the predefined criterion indicative of a temporal volatility comprises a volatility index, optionally wherein the volatility index comprises a vanishing value for static data, a value of 1 for data updated for each real time image, and/or a value of 1/m for data updated after m real time images, wherein m comprises a natural number.

5. The device (100) of any one of claims 1 to 4, wherein the second format from each of the multiple medical imaging sources comprises a signed distance field, SDF.

6. The device (100) of claim 5, wherein the combined final data set comprises a combined SDF.

7. The device (100) of any one of claims 1 to 6, wherein the combining unit (104) further comprises at least one filter applied to the received data from at least one or each of the multiple medical imaging sources, optionally wherein the at least one filter comprises an interpolation filter, an extrapolation filter, a three-dimensional Gaussian filter and/or a trilinear filter.

8. The device (100) of claim 7, wherein a size of the filter kernel increases with a predetermined property of the received data, optionally wherein the increase is linear.

9. The device (100) of any one of claims 1 to 8, wherein the real-time volumetric image data set comprises and/or is indicative of at least one of an ambient occlusion, AO, and a subsurface scattering, SSS, in relation to the part of the body based on the iteratively combined final data set.

10. The device (100) of any one of claims 1 to 9, wherein the multiple medical imaging sources are of heterogeneous type, wherein the medical imaging sources being of heterogenous type comprises at least one of a different classification in terms of
- latency,
- resolution, and/or
- accuracy,
among at least two of the multiple medical imaging sources.

11. The device (100) of any one of claims 1 to 10, wherein the receiving unit (102) is configured to receive data from multiple medical imaging sources comprising at least one of:
- an X-ray fluoroscopy device;
- an ultrasound device;
- a computed tomography, CT, device;
- a magnetic resonance imaging, MRI, device; and/or
- a medical instrument may be selected from the group consisting of at least:
- a catheter;
- a stent;
- a balloon;
- a wire; and/or
- a valve.

12. The device (100) of any one of claims 1 to 11 comprising a graphics processing unit, GPU, optionally wherein at least the step of iteratively combining the received data is performed by the GPU.

13. The device (100) of any one of claims 1 to 12, wherein the outputting unit (106) and/or the rendering unit (108) comprises a transfer function configured for outputting the real-time volumetric image data set and/or configured for rendering the volumetric image according to a predefined distance imaging scale, optionally wherein the predefined distance imaging scale comprises at least one of a color scale and a gray shades scale.

14. A computer-implemented method (300) of real-time volumetric imaging from multiple medical imaging sources of a part of a body during an interventional procedure, the multiple medical imaging sources comprising N+1 medical imaging sources of the part of the body with N greater or equal to two, the method (300) comprising or initiating the steps of:
- receiving (302) data from each of the multiple medical imaging sources regarding the part of the body, wherein the data from at least one or each of the multiple medical imaging sources are received (302) in a first format and converted into a predetermined second format, wherein the predetermined second format is universal for data received from any one of the multiple medical imaging sources;
- iteratively combining (304) the received data from each of the multiple medical imaging sources, wherein the data from the multiple medical imaging sources are combined (304) in the predetermined second format, and wherein iteratively combining (304) the data comprises
- ordering the data according to a predefined criterion indicative of a temporal volatility of the data based on the medical imaging source among the multiple medical imaging sources from which the data was received,
- combining the data of the two medical imaging sources among the multiple medical imaging sources with the two lowest temporal volatilities into a first intermediary combined data set,
- combining the first intermediary data set and the data from a third medical imaging source among the multiple medical imaging sources with the third lowest temporal volatility into a second intermediary combined data set, and
- in case of N greater or equal to three, combining the second intermediary data set and/or any further intermediary combined data set with the data from a fourth and/or any further medical imaging source with the next-lowest temporal volatility into a still further intermediary combined data set, and
- identifying the N-th combined data set of the iteration as a combined final data set; and
- outputting (306) a volumetric image data set regarding the part of the body based on the iteratively combined final data set;
wherein the receiving (302) step further comprises periodically updating the data from at least one or each of the multiple medical imaging sources, and wherein the combining (304) comprises repeating the iterative combining of the q-th data with the (q-2)-th intermediary data responsive to the periodic update of the p-th data, wherein p ≤ q ≤ N+1.

15. A computer program product comprising program code portions for performing the method of claim 14 when the computer program product is executed on one or more computing devices, optionally stored on a computer-readable recording medium.

## Patentansprüche

1. Vorrichtung (100) zur volumetrischen Echtzeitbildgebung von mehreren medizinischen Bildgebungsquellen eines Teils eines Körpers während eines interventionellen Vorgangs, die mehreren medizinischen Bildgebungsquellen umfassend N+1 medizinische Bildgebungsquellen des Teils des Körpers, wobei N größer oder gleich Zwei ist, die Vorrichtung (100) umfassend:
- eine Empfangseinheit (102), die dazu ausgelegt ist, Daten bezüglich des Teils des Körpers von jeder der mehreren medizinischen Bildgebungsquellen zu empfangen, wobei die Empfangseinheit (102) dazu ausgelegt ist, die Daten von mindestens einer oder jeder der mehreren medizinischen Bildgebungsquellen in einem ersten Format zu empfangen und die Daten in ein vorbestimmtes zweites Format umzuwandeln, wobei das vorbestimmte zweite Format universell für Daten ist, die von einer der mehreren medizinischen Bildgebungsquellen empfangen werden;
- eine Kombiniereinheit (104), die dazu ausgelegt ist, die empfangenen Daten von jeder der mehreren medizinischen Bildgebungsquellen iterativ zu kombinieren, wobei die Kombiniereinheit (104) dazu ausgelegt ist, die Daten von den mehreren medizinischen Bildgebungsquellen in dem vorbestimmten zweiten Format zu kombinieren, und wobei das iterative Kombinieren der Daten Folgendes umfasst:
- Ordnen der Daten gemäß einem vordefinierten Kriterium, das eine zeitliche Volatilität der Daten angibt, basierend auf der medizinischen Bildgebungsquelle unter den mehreren medizinischen Bildgebungsquellen, von denen die Daten empfangen wurden,
- Kombinieren der Daten der beiden medizinischen Bildgebungsquellen unter den mehreren medizinischen Bildgebungsquellen mit den beiden niedrigsten zeitlichen Volatilitäten zu einem ersten kombinierten Zwischendatensatz;
- Kombinieren des ersten Zwischendatensatzes und der Daten von einer dritten medizinischen Bildgebungsquelle unter den mehreren medizinischen Bildgebungsquellen mit der drittniedrigsten zeitlichen Volatilität zu einem zweiten kombinierten Zwischendatensatz, und
- in dem Fall, wo N größer oder gleich Drei ist, Kombinieren des zweiten Zwischendatensatzes und/oder eines weiteren kombinierten Zwischendatensatzes mit den Daten einer vierten und/oder einer weiteren medizinischen Bildgebungsquelle mit der nächstniedrigsten zeitlichen Volatilität in einen noch weiteren kombinierten Zwischendatensatz, und
- Identifizieren des N-ten kombinierten Datensatzes der Iteration als einen kombinierten endgültigen Datensatz; und
- eine Ausgabeeinheit (106), die dazu ausgelegt ist, einen volumetrischen Echtzeitbilddatensatz bezüglich des Teils des Körpers basierend auf dem iterativ kombinierten endgültigen Datensatz auszugeben;
wobei die Empfangseinheit (102) ferner dazu ausgelegt ist, die Daten von mindestens einer oder jeder der mehreren medizinischen Bildgebungsquellen periodisch zu aktualisieren, und wobei die Kombiniereinheit das iterative Kombinieren der q-ten Daten mit den (q-2)-ten Zwischendaten als Reaktion auf die periodische Aktualisierung der p-ten Daten wiederholt, wobei p ≤ q ≤ N+1.

2. Vorrichtung (100) nach Anspruch 1, ferner umfassend eine Rendering-Einheit (108), die dazu ausgelegt ist, ein volumetrisches Echtzeitbild des Teils des Körpers basierend auf dem volumetrischen Echtzeitbilddatensatz zu rendern.

3. Vorrichtung (100) nach Anspruch 1 oder 2, ferner umfassend eine Speichereinheit (206), die dazu ausgelegt ist, die empfangenen Daten, einen beliebigen der iterativ kombinierten Datensätze und/oder den volumetrischen Echtzeitbilddatensatz zu speichern.

4. Vorrichtung (100) nach einem der Ansprüche 1 bis 3, wobei das vordefinierte Kriterium, das eine zeitliche Volatilität angibt, einen Volatilitätsindex umfasst, wobei optional der Volatilitätsindex einen verschwindenden Wert für statische Daten, einen Wert von 1 für Daten, die für jedes Echtzeitbild aktualisiert werden, und/oder einen Wert von 1/m für Daten, die nach m Echtzeitbildern aktualisiert werden, umfasst, wobei m eine natürliche Zahl umfasst.

5. Vorrichtung (100) nach einem der Ansprüche 1 bis 4, wobei das zweite Format von jeder der mehreren medizinischen Bildgebungsquellen ein signiertes Abstandsfeld, SDF, umfasst.

6. Vorrichtung (100) nach Anspruch 5, wobei der kombinierte endgültige Datensatz ein kombiniertes SDF umfasst.

7. Vorrichtung (100) nach einem der Ansprüche 1 bis 6, wobei die Kombiniereinheit (104) ferner mindestens ein Filter umfasst, das auf die empfangenen Daten von mindestens einer oder jeder der mehreren medizinischen Bildgebungsquellen angewendet wird, wobei optional das mindestens ein Filter ein Interpolationsfilter, ein Extrapolationsfilter, ein dreidimensionales Gaußfilter und/oder ein trilineares Filter umfasst.

8. Vorrichtung (100) nach Anspruch 7, wobei eine Größe des Filterkernels mit einer vorbestimmten Eigenschaft der empfangenen Daten zunimmt, wobei die Zunahme optional linear ist.

9. Vorrichtung (100) nach einem der Ansprüche 1 bis 8, wobei der volumetrische Echtzeitbilddatensatz basierend auf dem iterativ kombinierten endgültigen Datensatz eine Umgebungsverdeckung, AO, und/oder eine Untergrundstreuung, SSS, in Bezug auf den Teil des Körpers umfasst und/oder angibt.

10. Vorrichtung (100) nach einem der Ansprüche 1 bis 9, wobei die mehreren medizinischen Bildgebungsquellen von heterogener Art sind, wobei die medizinischen Bildgebungsquellen heterogener Art mindestens eine von einer unterschiedlichen Klassifizierung in Bezug auf
- Latenz,
- Auflösung und/oder
- Genauigkeit,
unter mindestens zwei der mehreren medizinischen Bildgebungsquellen umfassen.

11. Vorrichtung (100) nach einem der Ansprüche 1 bis 10, wobei die Empfangseinheit (102) dazu ausgelegt ist, Daten von mehreren medizinischen Bildgebungsquellen zu empfangen, umfassend mindestens eines von Folgendem:
- eine Röntgendurchleuchtungsvorrichtung;
- eine Ultraschallvorrichtung;
- eine Computertomographie-Vorrichtung, CT-Vorrichtung;
- eine Magnetresonanztomographie-Vorrichtung, MRT-Vorrichtung; und/oder
- ein medizinisches Instrument kann aus der Gruppe ausgewählt werden, die mindestens besteht aus:
- einem Katheter;
- einem Stent;
- einem Ballon;
- einem Kabel; und/oder
- einem Ventil.

12. Vorrichtung (100) nach einem der Ansprüche 1 bis 11, umfassend eine Grafikverarbeitungseinheit, GPU, wobei optional mindestens der Schritt des iterativen Kombinierens der empfangenen Daten durch die GPU durchgeführt wird.

13. Vorrichtung (100) nach einem der Ansprüche 1 bis 12, wobei die Ausgabeeinheit (106) und/oder die Rendering-Einheit (108) eine Übertragungsfunktion umfasst, die zum Ausgeben des volumetrischen Echtzeitbilddatensatzes ausgelegt ist und/oder zum Rendern des volumetrischen Bildes gemäß einer vordefinierten Entfernungsbildgebungsskala ausgelegt ist, wobei die vordefinierte Entfernungsbildgebungsskala optional eine Farbskala und/oder eine Graustufenskala umfasst.

14. Computerimplementiertes Verfahren (300) zur volumetrischen Echtzeitbildgebung von mehreren medizinischen Bildgebungsquellen eines Teils eines Körpers während eines interventionellen Vorgangs, die mehreren medizinischen Bildgebungsquellen umfassend N+1 medizinische Bildgebungsquellen des Teils des Körpers, wobei N größer oder gleich Zwei ist, das Verfahren (300) umfassend die folgenden Schritte oder diese initiierend:
- Empfangen (302) von Daten von jeder der mehreren medizinischen Bildgebungsquellen bezüglich des Teils des Körpers, wobei die Daten von mindestens einer oder jeder der mehreren medizinischen Bildgebungsquellen in einem ersten Format empfangen (302) und in ein vorbestimmtes zweites Format umgewandelt werden, wobei das vorbestimmte zweite Format universell für Daten ist, die von einer der mehreren medizinischen Bildgebungsquellen empfangen werden;
- iteratives Kombinieren (304) der empfangenen Daten von jeder der mehreren medizinischen Bildgebungsquellen, wobei die Daten von den mehreren medizinischen Bildgebungsquellen in dem vorbestimmten zweiten Format kombiniert (304) werden, und wobei iteratives Kombinieren (304) der Daten Folgendes umfasst:
- Ordnen der Daten gemäß einem vordefinierten Kriterium, das eine zeitliche Volatilität der Daten angibt, basierend auf der medizinischen Bildgebungsquelle unter den mehreren medizinischen Bildgebungsquellen, von denen die Daten empfangen wurden,
- Kombinieren der Daten der beiden medizinischen Bildgebungsquellen unter den mehreren medizinischen Bildgebungsquellen mit den beiden niedrigsten zeitlichen Volatilitäten zu einem ersten kombinierten Zwischendatensatz;
- Kombinieren des ersten Zwischendatensatzes und der Daten von einer dritten medizinischen Bildgebungsquelle unter den mehreren medizinischen Bildgebungsquellen mit der drittniedrigsten zeitlichen Volatilität zu einem zweiten kombinierten Zwischendatensatz, und
- in dem Fall, wo N größer oder gleich Drei ist, Kombinieren des zweiten Zwischendatensatzes und/oder eines weiteren kombinierten Zwischendatensatzes mit den Daten einer vierten und/oder einer weiteren medizinischen Bildgebungsquelle mit der nächstniedrigsten zeitlichen Volatilität in einen noch weiteren kombinierten Zwischendatensatz, und
- Identifizieren des N-ten kombinierten Datensatzes der Iteration als einen kombinierten endgültigen Datensatz; und
- Ausgeben (306) eines volumetrischen Bilddatensatzes bezüglich des Teils des Körpers basierend auf dem iterativ kombinierten endgültigen Datensatz;
wobei der Schritt des Empfangens (302) ferner periodisches Aktualisieren der Daten von mindestens einer oder jeder der mehreren medizinischen Bildgebungsquellen umfasst und wobei das Kombinieren (304) das Wiederholen des iterativen Kombinierens der q-ten Daten mit den (q-2)-ten Zwischendaten als Reaktion auf die periodische Aktualisierung der p-ten Daten umfasst, wobei p ≤ q ≤ N+1.

15. Computerprogrammprodukt, umfassend Programmcodeabschnitte zum Durchführen des Verfahrens nach Anspruch 14, wenn das Computerprogrammprodukt auf einer oder mehreren Rechenvorrichtungen ausgeführt wird, die optional auf einem computerlesbaren Aufzeichnungsmedium gespeichert sind.

## Revendications

1. Dispositif (100) pour une imagerie volumétrique en temps réel à partir de multiples sources d'imagerie médicale d'une partie d'un corps pendant une procédure interventionnelle, les multiples sources d'imagerie médicale comprenant N + 1 sources d'imagerie médicale de la partie du corps, N étant supérieur ou égal à deux, le dispositif (100) comprenant :
- une unité de réception (102) configurée pour recevoir des données concernant la partie du corps en provenance de chacune des multiples sources d'imagerie médicale, dans lequel l'unité de réception (102) est configurée pour recevoir les données en provenance d'au moins une ou de chacune des multiples sources d'imagerie médicale dans un premier format et convertir les données dans un second format prédéterminé, dans lequel le second format prédéterminé est universel pour des données reçues en provenance de l'une quelconque des multiples sources d'imagerie médicale ;
- une unité de combinaison (104) configurée pour combiner de manière itérative les données reçues en provenance de chacune des multiples sources d'imagerie médicale, dans lequel l'unité de combinaison (104) est configurée pour combiner les données provenant des multiples sources d'imagerie médicale dans le second format prédéterminé, et dans lequel la combinaison itérative des données comprend
- le classement des données selon un critère prédéfini indiquant une volatilité temporelle des données en fonction de la source d'imagerie médicale parmi les multiples sources d'imagerie médicale en provenance de laquelle les données ont été reçues,
- la combinaison des données des deux sources d'imagerie médicale parmi les multiples sources d'imagerie médicale ayant les deux volatilités temporelles les plus faibles en un premier ensemble intermédiaire de données combinées,
- la combinaison du premier ensemble intermédiaire de données et des données provenant d'une troisième source d'imagerie médicale parmi les multiples sources d'imagerie médicale ayant la troisième volatilité temporelle la plus faible en un deuxième ensemble intermédiaire de données combinées, et
- dans le cas où N est supérieur ou égal à trois, la combinaison du deuxième ensemble intermédiaire de données et/ou d'un quelconque ensemble intermédiaire de données combinées supplémentaire avec les données provenant d'une quatrième et/ou d'une quelconque autre source d'imagerie médicale ayant la volatilité temporelle la plus faible suivante en encore un autre ensemble intermédiaire de données combinées, et
- l'identification du N-ième ensemble de données combinées de l'itération en tant qu'ensemble final de données combinées ; et
- une unité de sortie (106) configurée pour sortir un ensemble de données d'image volumétrique en temps réel concernant la partie du corps en fonction de l'ensemble final de données combinées de manière itérative ;
dans lequel l'unité de réception (102) est en outre configurée pour mettre à jour périodiquement les données provenant d'au moins une ou de chacune des multiples sources d'imagerie médicale, et dans lequel l'unité de combinaison répète la combinaison itérative des q-ièmes données avec les (q-2)-ièmes données intermédiaire en réponse à la mise à jour périodique des p-ièmes données, où p ≤ q ≤ N + 1.

2. Dispositif (100) selon la revendication 1, comprenant en outre une unité de rendu (108) configurée pour rendre une image volumétrique en temps réel de la partie du corps en fonction de l'ensemble de données d'image volumétrique en temps réel.

3. Dispositif (100) selon la revendication 1 ou 2, comprenant en outre une unité de stockage (206) configurée pour stocker les données reçues, l'un quelconque des ensembles de données combinées de manière itérative, et/ou l'ensemble de données d'image volumétrique en temps réel.

4. Dispositif (100) selon l'une quelconque des revendications 1 à 3, dans lequel le critère prédéfini indiquant une volatilité temporelle comprend un indice de volatilité, facultativement dans lequel l'indice de volatilité comprend une valeur de fuite pour des données statiques, une valeur de 1 pour des données mises à jour pour chaque image en temps réel, et/ou une valeur de 1/m pour des données mises à jour après m images en temps réel, dans lequel m comprend un nombre entier naturel.

5. Dispositif (100) selon l'une quelconque des revendications 1 à 4, dans lequel le second format provenant de chacune des multiples sources d'imagerie médicale comprend un champ de distance orientée, SDF.

6. Dispositif (100) selon la revendication 5, dans lequel l'ensemble final de données combinées comprend un SDF combiné.

7. Dispositif (100) selon l'une quelconque des revendications 1 à 6, dans lequel l'unité de combinaison (104) comprend en outre au moins un filtre appliqué aux données reçues en provenance d'au moins une ou de chacune des multiples sources d'imagerie médicale, éventuellement dans lequel l'au moins un filtre comprend un filtre d'interpolation, un filtre d'extrapolation, un filtre gaussien tridimensionnel et/ou un filtre trilinéaire.

8. Dispositif (100) selon la revendication 7, dans lequel la taille du noyau de filtre augmente avec une propriété prédéterminée des données reçues, facultativement dans lequel l'augmentation est linéaire.

9. Dispositif (100) selon l'une quelconque des revendications 1 à 8, dans lequel l'ensemble de données d'image volumétrique en temps réel comprend et/ou est révélateur d'une occlusion ambiante, AO, et/ou d'une diffusion subsuperficielle, SSS, en relation avec la partie du corps en fonction de l'ensemble final de données combinées de manière itérative.

10. Dispositif (100) selon l'une quelconque des revendications 1 à 9, dans lequel les multiples sources d'imagerie médicale sont de type hétérogène, dans lequel les sources d'imagerie médicale étant de type hétérogène comprennent au moins l'une d'une classification différente en termes de
- latence,
- résolution, et/ou
- précision,
parmi au moins deux des multiples sources d'imagerie médicale.

11. Dispositif (100) selon l'une quelconque des revendications 1 à 10, dans lequel l'unité de réception (102) est configurée pour recevoir des données en provenance de multiples sources d'imagerie médicale comprenant au moins l'un de :
- un dispositif de fluoroscopie à rayons X ;
- un dispositif à ultrasons ;
- un dispositif de tomodensitométrie, TDM ;
- un dispositif d'imagerie par résonance magnétique, IRM ; et/ou
- un instrument médical peut être choisi dans le groupe consistant au moins en :
- un cathéter ;
- une endoprothèse ;
- un ballonnet ;
- un fil ; et/ou
- une valve.

12. Dispositif (100) selon l'une quelconque des revendications 1 à 11 comprenant une unité de traitement graphique, GPU, facultativement dans lequel au moins l'étape de combinaison itérative des données reçues est effectuée par la GPU.

13. Dispositif (100) selon l'une quelconque des revendications 1 à 12, dans lequel l'unité de sortie (106) et/ou l'unité de rendu (108) comprennent une fonction de transfert configurée pour sortir l'ensemble de données d'image volumétrique en temps réel et/ou configurée pour rendre l'image volumétrique selon une échelle d'imagerie à distance prédéfinie, dans lequel facultativement l'échelle d'imagerie à distance prédéfinie comprend au moins une échelle de couleur et/ou une échelle de nuances de gris.

14. Procédé mis en œuvre par ordinateur (300) d'imagerie volumétrique en temps réel à partir de multiples sources d'imagerie médicale d'une partie d'un corps pendant une procédure interventionnelle, les multiples sources d'imagerie médicale comprenant N + 1 sources d'imagerie médicale de la partie du corps, N étant supérieur ou égal à deux, le procédé (300) comprenant ou déclenchant les étapes de :
- réception (302) de données provenant de chacune des multiples sources d'imagerie médicale concernant la partie du corps, les données en provenance d'au moins une ou de chacune des multiples sources d'imagerie médicale étant reçues (302) dans un premier format et converties en un second format prédéterminé, le second format prédéterminé étant universel pour les données reçues de l'une quelconque des multiples sources d'imagerie médicale ;
- combinaison itérative (304) des données reçues en provenance de chacune des multiples sources d'imagerie médicale, les données provenant des multiples sources d'imagerie médicale étant combinées (304) dans le second format prédéterminé, et la combinaison itérative (304) des données comprenant
- le classement des données selon un critère prédéfini indiquant une volatilité temporelle des données en fonction de la source d'imagerie médicale parmi les multiples sources d'imagerie médicale en provenance de laquelle les données ont été reçues,
- la combinaison des données des deux sources d'imagerie médicale parmi les multiples sources d'imagerie médicale ayant les deux volatilités temporelles les plus faibles en un premier ensemble intermédiaire de données combinées,
- la combinaison du premier ensemble intermédiaire de données et des données provenant d'une troisième source d'imagerie médicale parmi les multiples sources d'imagerie médicale ayant la troisième volatilité temporelle la plus faible en un deuxième ensemble intermédiaire de données combinées, et
- dans le cas où N est supérieur ou égal à trois, la combinaison du deuxième ensemble intermédiaire de données et/ou d'un quelconque ensemble intermédiaire de données combinées supplémentaire avec les données provenant d'une quatrième et/ou d'une quelconque autre source d'imagerie médicale ayant la volatilité temporelle la plus faible suivante en encore un autre ensemble intermédiaire de données combinées, et
- l'identification du N-ième ensemble de données combinées de l'itération en tant qu'ensemble final de données combinées ; et
- la sortie (306) d'un ensemble de données d'image volumétrique concernant la partie du corps en fonction de l'ensemble final de données combinées itérativement ; dans lequel l'étape de réception (302) comprend en outre la mise à jour périodique des données provenant d'au moins une ou de chacune des multiples sources d'imagerie médicale, et dans lequel la combinaison (304) comprend la répétition de la combinaison itérative des q-ièmes données avec les (q-2)-ièmes données intermédiaires en réponse à la mise à jour périodique des p-ièmes données, où p ≤ q ≤ N + 1.

15. Produit programme d'ordinateur comprenant des parties de code de programme permettant de réaliser le procédé selon la revendication 14 lorsque le produit programme d'ordinateur est exécuté sur un ou plusieurs dispositifs informatiques, facultativement stocké sur un support d'enregistrement lisible par ordinateur.
